# EUROPÄISCHE PATENTANMELDUNG

(11) **EP 2 865 411 A1**
(43) Veröffentlichungstag der Anmeldung: **29.04.2015**
(21) Anmeldenummer: 14003581.7
(22) Anmeldetag: 17.10.2014
(51) Int. Cl.: A61N 1/04, A61N 1/36, A61H 39/00

(54) **Stimulationsvorrichtung**

(30) Priorität: 28.10.2013 DE 102013017889
(71) Anmelder: cerboMed GmbH, 91052 Erlangen (DE)
(72) Erfinder: Zschaeck, Thomas, 90427 Nürnberg (DE); Bär, Stefan, 90556 Cadolzburg (DE); Hartlep, Andreas, 83607 Holzkirchen (DE); Frenkel, Wolf Gerhard, 72514 Inzigkofen-Engelswies (DE)
(74) Vertreter: Gosdin, Michael

(57) **Zusammenfassung**

Die Erfindung betrifft eine Stimulationsvorrichtung (1) zur Aufbringung eines transkutanen elektrischen Stimulationsreizes auf die Oberfläche eines Abschnittes des Ohrs, die ein Halteelement (2) sowie mindestens eine Elektrode (3, 4) in einem Elektrodenträger (5) aufweist, wobei das Halteelement (2) eine Linearführung (6) aufweist, wobei am Halteelement (2) ein Auflageteil (8) angeordnet ist, wobei eine Haltestange (7) eine erste Achse (A) aufweist, wobei die Haltestange (7) von einer ersten Position (P1), die an die Linearführung (6) angrenzt, zu einer zweiten Position (P2), die an den Elektrodenträger (5) angrenzt, verläuft, wobei das Auflageteil (8) mit einer Auflagefläche (9) am Ohr eine erste Ebene (E0) definiert, auf der eine zweite Achse (B) senkrecht steht, wobei die erste Achse (A) und die zweite Achse (B) eine Mittenebene (E1) aufspannen. Um durch verbesserte Platzierung der Elektroden auf der Hautoberfläche des Ohrs eine bessere Stimulation zu ermöglichen, sieht die Erfindung vor, dass die Haltestange (7) an der ersten Position (P1) in der Mittenebene (E1) liegt, dass sich die Haltestange (7) vom Verlauf von der ersten Position (P1) hin zur zweiten Position (P2) in einem ersten Stangenabschnitt (10) bis zu einem Umkehrpunkt (PU) von der Mittenebene (E1) entfernt, dass sich die Haltestange (7) vom Verlauf vom Umkehrpunkt (PU) hin zur zweiten Position (P2) in einem zweiten Stangenabschnitt (11) hin zur Mittenebene (E1) bewegt, um bei Erreichen der zweiten Position (P2) in der Mittenebene (E1) zu liegen.

## Beschreibung

Die Erfindung betrifft eine Stimulationsvorrichtung zur Aufbringung eines transkutanen elektrischen Stimulationsreizes auf die Oberfläche eines Abschnittes des menschlichen Ohrs, die ein am oder im Ohr anbringbares Halteelement sowie mindestens eine Elektrode aufweist, die in einem Elektrodenträger angeordnet ist, wobei das Halteelement eine Linearführung aufweist, in der eine Haltestange in Richtung einer Längsachse des Halteelements linear verschieblich angeordnet ist, wobei an einem von der Linearführung beabstandeten axialen Ende der Haltestange der Elektrodenträger angeordnet ist, wobei am Halteelement ein Auflageteil angeordnet oder angeformt ist, das zur Auflage auf einem Abschnitt des Ohrs ausgebildet ist, wobei die Haltestange eine erste Achse aufweist, die im Bereich der Linearführung und parallel zur Längsachse in der Haltstange verläuft, wobei die Haltestange von einer ersten Position, die an die Linearführung angrenzt, zu einer zweiten Position, die an den Elektrodenträger angrenzt, verläuft, wobei das Auflageteil mit einer Auflagefläche am Ohr eine erste Ebene definiert, auf der eine zweite Achse senkrecht steht, wobei die erste Achse und die zweite Achse eine Mittenebene aufspannen.

Eine gattungsgemäße Stimulationsvorrichtung ist aus der DE 10 2012 014 764 A1 bekannt. Eine solche Stimulationsvorrichtung hat sich bewährt, um mittels der Methode der transkutanen elektrischen Nervenstimulation Impulsströme verschiedener Stromformen, Amplituden, Impulsdauern und Frequenzen durch die Haut hindurch an verschiedenen Nerven zu applizieren und so verschiedene Krankheiten zu behandeln.

Bei der vorbekannten Stimulationsvorrichtung geht die Haltestange - aus dem Bereich der Linearführung kommend - in einen blattfederartig ausgebildeten Bereich über, der gegenüber dem Abschnitt im Bereich der Linearführung zwar abgewinkelt ist, jedoch linear zum Elektrodenträger verläuft.

Es hat sich herausgestellt, dass bei dieser vorbekannten Lösung nicht immer gewährleistet ist, dass der Elektrodenträger mit den Elektroden in ergonomisch günstiger Weise auf der Hautoberfläche platziert werden kann und gleichzeitig ein hoher Tragekomfort gewährleistet ist.

Der Erfindung liegt daher die **Aufgabe** zugrunde, eine Stimulationsvorrichtung der gattungsgemäßen Art so fortzubilden, dass der genannte Nachteil überwunden wird. Es soll also eine Weiterbildung dahingehend vorgeschlagen werden, dass durch eine verbesserte Platzierung der Elektroden auf der Hautoberfläche des Ohrs eine bessere Stimulation ermöglicht wird, wobei gleichzeitig ein hohes Niveau für den Tragekomfort der Stimulationsvorrichtung erreicht werden soll.

Die **Lösung** dieser Aufgabe durch die Erfindung ist dadurch gekennzeichnet, dass die Haltestange an der ersten Position in der oder nahe der Mittenebene liegt, dass sich die Haltestange vom Verlauf von der ersten Position hin zur zweiten Position in einem ersten Stangenabschnitt bis zu einem Umkehrpunkt von der Mittenebene entfernt, dass sich die Haltestange vom Verlauf vom Umkehrpunkt hin zur zweiten Position in einem zweiten Stangenabschnitt hin zur Mittenebene bewegt, um bei Erreichen der zweiten Position in der oder nahe der Mittenebene zu liegen.

Die Haltestange verläuft - im Unterschied zur vorbekannten, oben diskutierten Lösung - also von der Linearführung zum Elektrodenträger nicht linear, sondern weicht seitlich aus. Damit kann der Elektrodenträger in verbesserter Weise und ergonomisch günstig zu der Stelle der Hautoberfläche des Ohrs, insbesondere in den Bereich der Cymba conchae, geführt werden, die mit einem transkutanen Stimulationsstrom beaufschlagt werden soll.

Durch den im Raum doppelt-S-förmig geschwungenen Verlauf der Haltestange verfügt diese über eine Federwirkung mit im Vergleich zu einem geraden Verlauf relativ flacher Weg-(Gegen-)Kraft- bzw. Druckkurve. Dadurch werden bei dennoch optimalem Elektrodenkontakt Druckstellen an der Haut des Ohres vermieden. Diese Eigenschaft kann auch durch entsprechende Materialwahl begünstigt werden, so dass Druckstellen in der Cymba conchae vermieden werden können.

Die Haltestange fällt dabei bevorzugt zwischen der ersten Position zur zweiten Position - gemessen in Richtung der zweiten Achse - um einen Höhenversatz in Richtung auf die erste Ebene ab. Der Abstand der Haltestange im Umkehrpunkt von der Mittenebene beträgt dabei bevorzugt zwischen 30 % und 60 % des Höhenversatzes.

Die Haltestange weist bevorzugt an der ersten Position eine Tangente auf, die in Richtung der ersten Achse weist. Indes ist weiter vorzugsweise vorgesehen, dass die Haltestange beim Einlauf in den Elektrodenträger an der zweiten Position eine Tangente aufweist, die in der Ansicht gesehen in Richtung der zweiten Achse einen Winkel zur ersten Achse einschließt, der zwischen 50° und 80° beträgt.

Die Haltestange weist bevorzugt über den gesamten Verlauf von der ersten Position bis zur zweiten Position einen geschwungenen, knickfreien Verlauf auf.

Die Haltestange besteht bevorzugt zumindest im Bereich zwischen der ersten Position bis zur zweiten Position aus einem elastischen Material, insbesondere aus Kunststoff.

Die Stromversorgung der mindestens einen Elektrode kann vom Halteelement aus über ein Kabel erfolgen, das separat zwischen dem Halteelement und dem Elektrodenträger verläuft.

Die Haltestange kann dabei aus zwei Teilen bestehen, nämlich aus einem ersten (geraden) Teil, der in der Linearführung verläuft, und einem zweiten (S-förmig geschwungen ausgeführten) Teil, der zwischen der ersten Position und der zweiten Position verläuft. Die beiden Teile der Haltestange sind dabei bevorzugt über eine formschlüssige Verbindung miteinander verbunden.

Mit der vorgeschlagenen Lösung kann in sehr vorteilhafter Weise erreicht werden, dass die Stimulationsvorrichtung mit der Auflagefläche des Auflageteils so im Ohr platziert wird, dass das Auflageteil unter dem Tragus im Cavum conchae aufliegt, der Elektrodenträger mit seinen Elektroden indes ergonomisch günstig und mit hohem Tragekomfort zur Auflage im Bereich der Cymba conchae geführt wird. Zur diesbezüglichen Ausgestaltung wird ausdrücklich auf die DE 10 2012 014 764 A1 der Anmelderin Bezug genommen, wo hierzu Details erläutert sind.

Die Stimulationsvorrichtung kann hinsichtlich ihrer detaillierten Ausgestaltung mit diversen Elementen ausgeführt sein, wie sie in der DE 10 2010 054 165 B3 und in der bereits oben genannten DE 10 2012 014 764 A1 beschrieben sind. Auf diese beiden vorbekannten Ausgestaltungen der Anmelderin wird ausdrücklich Bezug genommen. Dies gilt insbesondere hinsichtlich der Ausgestaltung und Anordnung der Elektroden am Elektrodenträger sowie für die Ausgestaltung der Linearführung und des Auflageteils.

Wie sich aus dem Vorgesagten ergibt, ist unter dem Begriff der "Haltestange" keineswegs lediglich ein sich linear erstreckendes Element zu verstehen; lediglich im Bereich der Linearführung liegt eine lineare Gestaltung der Haltestange vor. Ansonsten verläuft die Haltestange zwischen der Linearführung und dem Elektrodenträger in der erläuterten S-förmig geschwungenen Weise.

Die Haltestange kann beispielsweise eine Seele aus elastischem Material, insbesondere Metall (besonders bevorzugt Federstahl) aufweisen, wobei diese Seele von einem Ummantelungsmaterial, insbesondere von einem biokompatiblen Kunststoffmaterial umgeben ist; hierfür hat sich besonders Silikon, Polyamid, Polypropylen oder Polyurethan bewährt.

Auch der Elektrodenträger kann zumindest teilweise aus elastischem Material, insbesondere aus biokompatiblem Kunststoffmaterial, besonders bevorzugt aus Silikon, aus Polyethylen, aus Polypropylen oder aus Polyurethan, bestehen.

Der Elektrodenträger weist, insbesondere als Elektrodenkopf ausgebildet, bevorzugt mindestens eine Stimulationselektrode und mindestens eine Referenzelektrode auf.

Die Elektroden sind bevorzugt Metallelektroden, insbesondere Titanelektroden. Sie weisen bevorzugt die Form eines Kugelabschnitts oder eines Abschnitts eines Ellipsoids auf.

Die Teile der Stimulationsvorrichtung sind bevorzugt - soweit Hautkontakt gegeben ist - auswechselbar und bestehen aus einem weichen Material, wobei speziell an ein Elastomermaterial gedacht ist, insbesondere an Silikon oder an ein Material, das Silikon aufweist. Im Falle des Elektrodenträgers kann die leichte Auswechselbarkeit dadurch erreicht werden, dass das Kabel am Elektrodenträger einsteckbar gestaltet ist.

Des weiteren haben sich folgende Weiterbildungen des vorgeschlagenen Konzepts als sehr vorteilhaft erwiesen:
Die beiden Teile der Haltestange können aus Materialien unterschiedlicher Elastizität und somit bei einer Verformung mit unterschiedlicher Rückstellkraft bestehen.

Sehr günstig ist ferner ein Konzept, wonach ein Satz mit mehreren zweiten Teilen der Haltestange vorgesehen ist. Diese zweiten Teile unterscheiden sich in ihrer Geometrie und/oder im verwendeten Material und somit in ihrer Feder- bzw. Rückstellkraft, was durch die Ausformung bzw. entsprechende Materialwahl gezielt beeinflusst werden kann. Ein wesentlicher geometrischer Einflussfaktor ist insbesondere die Größe der Radien der doppelt-geschwungen ausgebildeten Haltestange. Der Vorteil ist hier, dass ein optimales zweites Teil der Haltestange vom Arzt oder Anwender bzw. Patienten ausgewählt und verwendet werden kann. Die Haltestange kann so der Kontur des Ohres und der Empfindlichkeit des Patienten angepasst werden.

Das Kabel bzw. die elektrisch leitenden Litzen können zumindest abschnittsweise innerhalb der Haltestange verlaufen.

Vorgesehen kann auch werden, dass zumindest innerhalb des zweiten Teils der Haltestange eine Seele aus biegsamem Material (bevorzugt aus Metall) verläuft, mittels der die Haltestange von einer ersten in eine zweite Position gebogenen werden kann. Damit kann der Anwender der Vorrichtung die Haltestange auf ein individuelles Relief des jeweiligen Ohrs zurecht biegen.

In der Zeichnung ist ein Ausführungsbeispiel der Erfindung dargestellt. Es zeigen:
- Fig. 1: die Draufsicht auf eine Stimulationsvorrichtung, die in ein Ohr eines Menschen eingesetzt ist,
- Fig. 2: in perspektivischer Darstellung das Halteelement der Stimulationsvorrichtung mit an diesem angeformten Auflageteil und
- Fig. 3: in perspektivischer Darstellung einen Teil des Halteelements der Stimulationsvorrichtung.

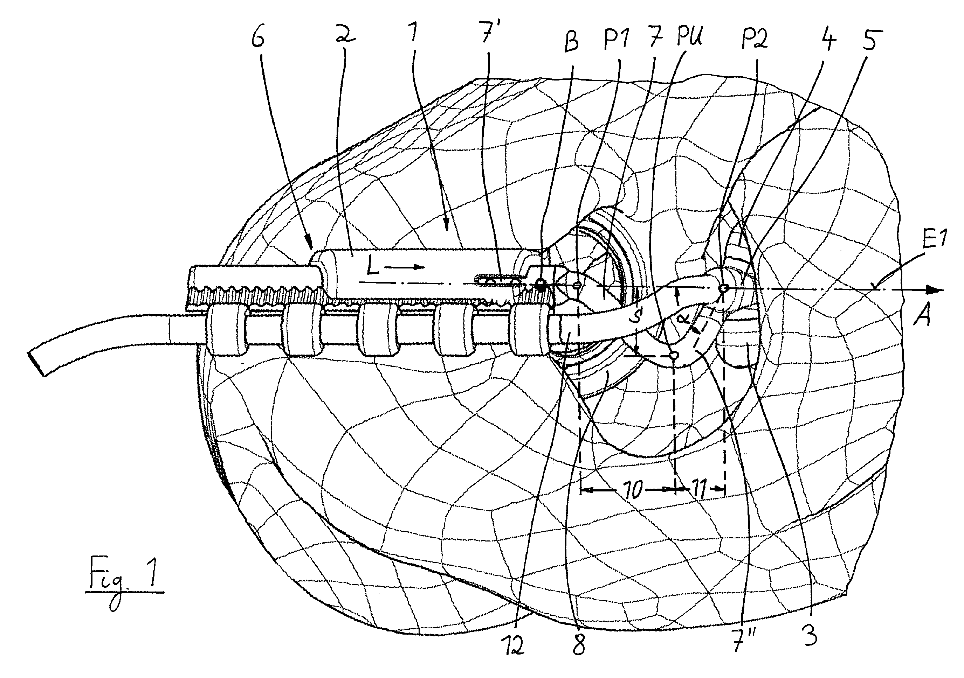

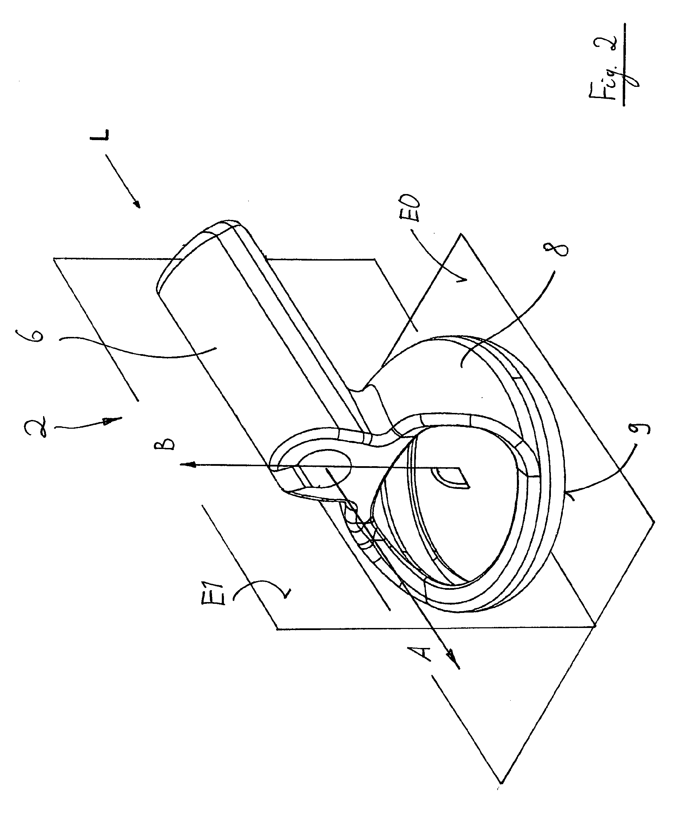

In Fig. 1 ist eine Stimulationsvorrichtung 1 zu sehen, die in ein teilweises dargestelltes Ohr eines Patienten eingesetzt ist. Mit der Stimulationsvorrichtung wird eine transkutane Nervenstimulation vorgenommen. Zentrales Teil der Stimulationsvorrichtung 1 ist ein Halteelement 2, das in Fig. 2 als Einzelteil dargestellt ist. Das Halteelement 2 hat eine Linearführung 6, in der eine Haltestange 7 (s. Fig. 1) in Richtung einer Längsachse L linear verschieblich angeordnet ist.

Am einen axialen Ende der Haltestange 7 ist ein Elektrodenträger 5 angeordnet, der zwei Elektroden 3 und 4 trägt, nämlich eine Stimulationselektrode und eine Referenzelektrode.

Am Halteelement 2 ist weiterhin ein Auflageteil 8 angeformt, das eine Auflagefläche 9 hat (s. Fig. 2), die zur Auflage im Cavum conchae des Ohrs ausgebildet ist. Liegt die Stimulationsvorrichtung 1 in dieser Weise im Ohr an - s. hierzu Fig. 1 -, reicht die Haltestange 7 mit dem an ihrem Ende angeordneten Elektrodenträger 5 in die Cymba conchae hinein, so dass hier die beiden Elektroden 3 und 4 auf der Haut aufliegen und transkutan stimulieren können.

Zu diesbezüglichen Einzelheiten wird ausdrücklich auf die DE 10 2010 054 165 B3 und die DE 10 2012 014 764 A1 Bezug genommen, wo sich hierzu detaillierte Erläuterungen befinden.

Wie in der Zusammenschau der Figuren zu erkennen ist, hat die Haltestange 7 in dem Bereich, in dem sie in der Linearführung 6 verläuft, einen geradlinigen Verlauf, der durch eine erste Achse A gekennzeichnet ist, die parallel zur Längsachse L verläuft bzw. mit dieser identisch ist. Betrachtet man das Auflageteil 8 mit der im Cavum conchae aufliegenden Auflagefläche 9, ist festzustellen, dass sich eine erste Ebene E0 definieren lässt, die sich durch die Auflagefläche 9 ergibt. Auf dieser Ebene E0 steht eine zweite Achse B senkrecht. Die beiden Achsen A und B spannen eine Mittenebene E1 auf.

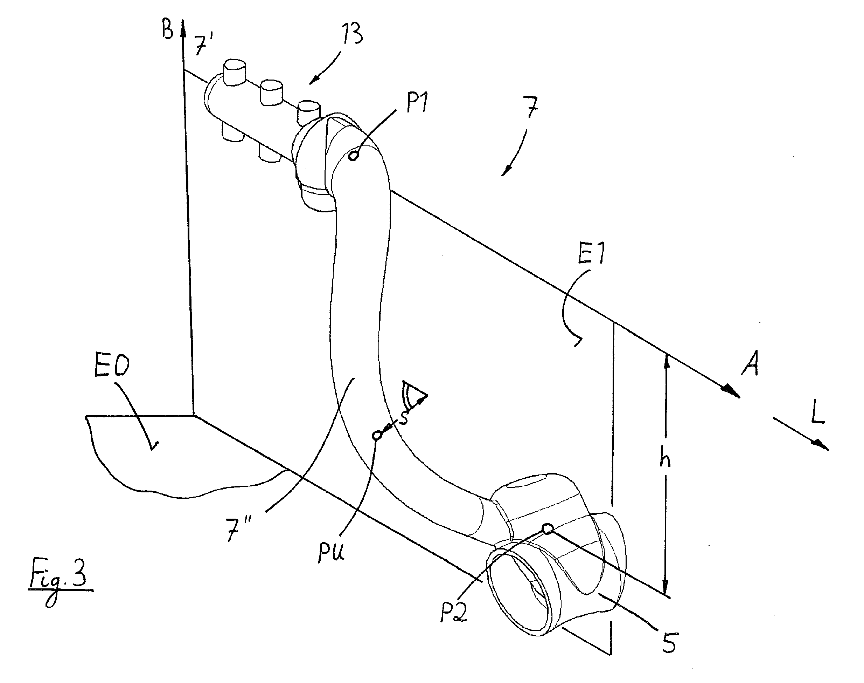

Wie sich mit Blick auf diese Definitionen ergibt, ist vorgesehen, dass die Haltestange 7 von einer ersten Position P1 (s. Fig. 1 und Fig. 3), die an die Linearführung 6 angrenzt, zu einer zweiten Position P2, die an den Elektrodenträger 5 angrenzt, verläuft. Die Haltestange 7 liegt an der ersten Position P1 in der Mittenebene E1 bzw. ist zumindest nahe an dieser angeordnet. Die Haltestange 7 entfernt sich dann vom Verlauf von der ersten Position P1 hin zur zweiten Position P2 in einem ersten Stangenabschnitt 10 bis zu einem Umkehrpunkt PU von der Mittenebene E1, d. h. der kürzeste Abstand der Haltestange 7 von der Ebene E1 nimmt ständig zu. Indes bewegt sich die Haltestange 7 vom Verlauf vom Umkehrpunkt PU hin zur zweiten Position P2 in einem zweiten Stangenabschnitt 11 wieder hin zur Mittenebene E1. In der zweiten Position P2 wird die Mittenebene E1 wieder erreicht bzw. die Haltestange 7 liegt hier zumindest wieder nahe an der Mittenebene E1. Der maximale Abstand, den die Haltestange 7 am Umkehrpunkt PU von der Mittenebene E1 hat, ist mit s bezeichnet.

Wie sich weiter ergibt, verläuft die Haltestange 7 zwischen den Positionen P1 und P2 nicht nur in der erläuterten Weise geschwungen von der Mittenebene E1 weg und dann wieder zu selbiger zurück, sondern von der Höhe der Linearführung 6 wird der Elektrodenträger 5 auch um einen Höhenversatz h abwärts in Richtung der Hautoberfläche geführt.

Wie weiter gesehen werden kann, weist die Haltestange 7 an der ersten Position P1 eine Tangente auf, die in Richtung der ersten Achse A weist. Indes ist zu sehen - hierzu wird auf Fig. 1 hingewiesen -, dass die Haltestange 7 beim Einlauf in den Elektrodenträger 5 an der zweiten Position P2 eine Tangente aufweist, die in der Draufsicht gemäß Fig. 1 einen Winkel α zur ersten Achse A einschließt; dieser Winkel liegt im Ausführungsbeispiel bei ca. 55°, bevorzugt liegt er zwischen 50° und 80°.

Die Haltestange 7 ist vorliegend zweiteilig ausgebildet. Ein erstes Teil 7' ist der Bereich, der in der Linearführung 6 verläuft. Ein zweiter Teil 7" ist indes der Teil (dargestellt in Fig. 3), der den geschwungenen, S-förmigen Verlauf aufweist und die Verbindung zwischen der ersten Position P1 und der zweiten Position P2 bildet.

Die Haltestange 7 ist aus diesen beiden Teilen 7' und 7" hergestellt, die separat gefertigt und dann über eine formschlüssige Verbindung 13 miteinander verbunden sind. Hierzu weist im Ausführungsbeispiel das Teil 7" mehrere sich radial von dem Stangengrundkörper erstreckende zylindrische Noppen auf, die in korrespondierende Ausnehmungen im Teil 7' (nicht dargestellt) eingreifen; der Abschnitt des Teils 7', in den das Teil 7" eingreift, ist hierzu als Hülse ausgebildet, in die das axiale Ende des Teils 7" eintreten kann. Die Teile 7' und 7" können so zusammengesteckt und dadurch fest verbunden werden.

Bei Wahl eines entsprechenden Materials, das zugleich formstabil genug ist, um problemlos in der Linearführung zu laufen und dennoch die oben beschriebene Federwirkung aufweist, kann die Haltestange jedoch auch einstückig ausgebildet und hergestellt sein.

Die Stromversorgung der Elektroden 3, 4 erfolgt über ein Kabel 12. Details hierzu sind wiederum in der DE 10 2010 054 165 B3 und der DE 10 2012 014 764 A1 enthalten, so dass insoweit hierauf Bezug genommen wird.

Die mechanische Verbindung des Elektrodenträgers 5 samt Elektroden 3, 4 mit dem Halteelement 2 wird somit durch die Haltestange 7 und insbesondere deren Teil 7" hergestellt, während die elektrische Verbindung durch das Kabel 12 bewerkstelligt wird, das im Vergleich mit der Haltestange 7 nachgiebig und biegeschlaff ist.

### Bezugszeichenliste:

- 1: Stimulationsvorrichtung
- 2: Halteelement
- 3: Elektrode
- 4: Elektrode
- 5: Elektrodenträger
- 6: Linearführung
- 7: Haltestange
- 7': erster Teil der Haltestange
- 7": zweiter Teil der Haltestange
- 8: Auflageteil
- 9: Auflagefläche
- 10: erster Stangenabschnitt
- 11: zweiter Stangenabschnitt
- 12: Kabel
- 13: formschlüssige Verbindung

- L: Längsachse
- A: erste Achse
- B: zweite Achse

- P1: erste Position
- P2: zweite Position
- PU: Umkehrpunkt

- E0: erste Ebene
- E1: Mittenebene

- h: Höhenversatz
- s: Abstand von der Mittenebene
- α: Winkel

## Patentansprüche

1. Stimulationsvorrichtung (1) zur Aufbringung eines transkutanen elektrischen Stimulationsreizes auf die Oberfläche eines Abschnittes des menschlichen Ohrs, die ein am oder im Ohr anbringbares Halteelement (2) sowie mindestens eine Elektrode (3, 4) aufweist, die in einem Elektrodenträger (5) angeordnet ist,
wobei das Halteelement (2) eine Linearführung (6) aufweist, in der eine Haltestange (7) in Richtung einer Längsachse (L) des Halteelements (2) linear verschieblich angeordnet ist,
wobei an einem von der Linearführung (6) beabstandeten axialen Ende der Haltestange (7) der Elektrodenträger (5) angeordnet ist,
wobei am Halteelement (2) ein Auflageteil (8) angeordnet oder angeformt ist, das zur Auflage auf einem Abschnitt des Ohrs ausgebildet ist,
wobei die Haltestange (7) eine erste Achse (A) aufweist, die im Bereich der Linearführung (6) und parallel zur Längsachse (L) in der Haltstange (7) verläuft,
wobei die Haltestange (7) von einer ersten Position (P1), die an die Linearführung (6) angrenzt, zu einer zweiten Position (P2), die an den Elektrodenträger (5) angrenzt, verläuft,
wobei das Auflageteil (8) mit einer Auflagefläche (9) am Ohr eine erste Ebene (E0) definiert, auf der eine zweite Achse (B) senkrecht steht,
wobei die erste Achse (A) und die zweite Achse (B) eine Mittenebene (E1) aufspannen,
**dadurch gekennzeichnet,**
**dass** die Haltestange (7) an der ersten Position (P1) in der oder nahe der Mittenebene (E1) liegt,
**dass** sich die Haltestange (7) vom Verlauf von der ersten Position (P1) hin zur zweiten Position (P2) in einem ersten Stangenabschnitt (10) bis zu einem Umkehrpunkt (PU) von der Mittenebene (E1) entfernt,
**dass** sich die Haltestange (7) vom Verlauf vom Umkehrpunkt (PU) hin zur zweiten Position (P2) in einem zweiten Stangenabschnitt (11) hin zur Mittenebene (E1) bewegt, um bei Erreichen der zweiten Position (P2) in der oder nahe der Mittenebene (E1) zu liegen.

2. Stimulationsvorrichtung nach Anspruch 1, **dadurch gekennzeichnet, dass** die Haltestange (7) zwischen der ersten Position (P1) zur zweiten Position (P2), gemessen in Richtung der zweiten Achse (B), um einen Höhenversatz (h) in Richtung auf die erste Ebene (E0) abfällt.

3. Stimulationsvorrichtung nach Anspruch 2, **dadurch gekennzeichnet, dass** der Abstand (s) der Haltestange (7) im Umkehrpunkt (PU) von der Mittenebene (E1) zwischen 30 % und 60 % des Höhenversatzes (h) beträgt.

4. Stimulationsvorrichtung nach einem der Ansprüche 1 bis 3, **dadurch gekennzeichnet, dass** die Haltestange (7) an der ersten Position (P1) eine Tangente aufweist, die in Richtung der ersten Achse (A) weist.

5. Stimulationsvorrichtung nach einem der Ansprüche 1 bis 4, **dadurch gekennzeichnet, dass** die Haltestange (7) beim Einlauf in den Elektrodenträger (5) an der zweiten Position (P2) eine Tangente aufweist, die in der Ansicht gesehen in Richtung der zweiten Achse (B) einen Winkel (α) zur ersten Achse (A) einschließt, der zwischen 50° und 80° beträgt.

6. Stimulationsvorrichtung nach einem der Ansprüche 1 bis 5, **dadurch gekennzeichnet, dass** die Haltestange (7) über den gesamten Verlauf von der ersten Position (P1) bis zur zweiten Position (P2) einen geschwungenen, knickfreien Verlauf aufweist.

7. Stimulationsvorrichtung nach einem der Ansprüche 1 bis 6, **dadurch gekennzeichnet, dass** die Haltestange (7) zumindest im Bereich zwischen der ersten Position (P1) bis zur zweiten Position (P2) aus einem elastischen Material besteht, insbesondere aus Kunststoff.

8. Stimulationsvorrichtung nach einem der Ansprüche 1 bis 7, **dadurch gekennzeichnet, dass** die Stromversorgung der mindestens einen Elektrode (3, 4) vom Halteelement (2) aus über ein Kabel (12) erfolgt, das separat zwischen dem Halteelement (2) und dem Elektrodenträger (5) verläuft.

9. Stimulationsvorrichtung nach einem der Ansprüche 1 bis 8, **dadurch gekennzeichnet, dass** die Haltestange (7) aus zwei Teilen besteht, nämlich aus einem ersten Teil (7'), der in der Linearführung (6) verläuft, und einem zweiten Teil (7"), der zwischen der ersten Position (P1) und der zweiten Position (P2) verläuft.

10. Stimulationsvorrichtung nach Anspruch 9, **dadurch gekennzeichnet, dass** die beiden Teile (7', 7") der Haltestange (7) über eine formschlüssige Verbindung (13) miteinander verbunden sind.
